(19) Europäisches Patentamt
European Patent Office
Office européen des brevets

(11) Publication number: **0 173 567**
**A2**

# (12) EUROPEAN PATENT APPLICATION

(21) Application number: 85306118.2

(22) Date of filing: 29.08.85

(51) Int. Cl.[4]: C 08 F 2/50, A 61 K 6/08

(30) Priority: 30.08.84 US 645733

(43) Date of publication of application: 05.03.86 Bulletin 86/10

(84) Designated Contracting States: AT BE CH DE FR GB IT LI NL SE

(71) Applicant: JOHNSON & JOHNSON DENTAL PRODUCTS COMPANY, 20 Lake Drive CN 7060, East Windsor New Jersey 08520 (US)

(72) Inventor: Ying, Lincoln, 266 Holland Court, Bridgewater New Jersey 08807 (US)

(74) Representative: Jones, Alan John et al, CARPMAELS & RANSFORD 43 Bloomsbury Square, London, WC1A 2RA (GB)

## (54) Method of treating teeth with certain photopolymerizable compositions including catalyst comprising an acylphosphine oxide.

(57) A method of treating teeth with a photopolymerizable composition comprising at least one olefinically unsaturated monomer and an acylphosphine oxide catalyst.

EP 0 173 567 A2

METHOD OF TREATING TEETH WITH CERTAIN PHOTOPOLYMERIZABLE COMPOSITIONS INCLUDING CATALYST COMPRISING AN ACYLPHOSPHINE OXIDE

The invention relates to a method of treating teeth with a photopolymerizable composition including at least one olefinically unsaturated compound and a photosensitive catalyst containing an acylphosphine oxide.

## Background of the Invention

The use of photopolymerizable compositions for end use applications such as pit and fissure sealants, bonding agents, and dental composites is of commercial interest because such compositions can be marketed as one-package systems and therefore do not need to be mixed at the point of use such as is the case with the usual peroxide curable composition which must be mixed with an activator just before use.

This invention is directed to a method of treating teeth with a photopolymerizable dental restorative composition containing an acylphosphine oxide catalyst system that is highly effective when employed in conjunction with visible light, and which exhibits rapid cure rate and excellent properties after cure.

Heretofore, the best photopolymerizable dental compositions known to the inventor herein employ a catalyst system including a mixture of camphoroquinone and benzil. The acylphosphine oxide catalyst system of this invention provides ultimate properties similar to those which can be obtained by the camphoroquinone/benzil system, but with the added desireable feature of providing an optically clear resin as opposed to the light orange-yellow resin

obtained with the camphoroquinone/benzil system. This is an advantage in the development of dental compositions having optimum cosmetic properties.

## Brief Summary of the Invention

The invention provides a composition comprising at least one olefinically unsaturated compound and a catalyst comprising an acylphosphine oxide for treating teeth by its application to a prepared tooth.

## The Prior Art

Photopolymerizable dental compositions are known. For instance, they are described in Dart et al., U.S. Patent No. 4,071,424, in Schmitt et al., U.S. Patent No. 4,189,365, Jochum et al., U.S. Patent No. 4,351,853, Orlowski et al., U.S. Patent No. 4,427,799, Kemper, U.S. Patent No. 4,261,879, and Kumar, U.S. Patent No. 4,500,657, and in published European application Nos. 0, 102,199 and 0,132,959.

Acylphosphine oxides are described as being useful as photosensitizers for molding, impregnating, and coating compositions. For instance, see Hesse et al., U.S. Patent No. 4,265,723. In Lechtken et al., U.S. Patent No. 4,292,152, it is disclosed that acylphosphine oxides can be used in conjunction with tertiary amines as photosensitizing catalysts in photopolymerizable recording compositions useful in the preparation of printing plates, relief plates, and photoresists. The use of acylphosphine oxide as photosensitizing catalysts in similar applications is disclosed in Lechtin et al., U.S. Patent Nos. 4,298,738, 4,324,744, and 4,385,109, Lehner et al., U.S. Patent No. 4,421,840, Hesse et al., U.S. Patent No. 4,425,287, and Heinz et al., U.S. Patent No. 4,430,417.

## Detailed Description of the Invention

The photopolymerizable dental composition used in the invention includes at least one olefinically unsaturated compound. Such materials are known in the art, and need to be illustrated by only a few examples. It is preferred that the olefinically unsaturated compound be an acrylic or methacrylic ester, and particularly, a compound having two or more acrylic or methacrylic ester groups because the polyfunctional acrylic esters exhibit less shrinkage upon polymerization than do the monofunctional acrylic esters, and also provide cross-linking. Specific types of acrylic esters that are useful include alkane diol acrylates or methacrylates such as the $C_4$-$C_{12}$ alkane diol acrylates or methacrylates, e.g., 1,10-decamethylene diol dimethacrylate and 1,6-hexamethylene diol dimethacrylate; the polyalkylene glycol acrylates or methacrylates, such as triethylene glycol dimethacrylate and tetraethylene glycol dimethacrylate; bisphenol-A acrylate or methacrylate esters; alkoxylated bisphenol-A acrylate or methacrylate, e.g., ethoxylated bisphenol-A dimethacrylate; bisphenol-A diglycidyl dimethacrylate ("Bis-GMA"); and the like. Other multifunctional acrylic or methacrylic esters that can be used include methacrylate-terminated polyurethanes, trimethylolpropane trimethacrylate or triacrylate, and the like.

In certain preferred aspects of the invention, a monofunctional monomer (i.e., a monomer containing only one olefinically unsaturated group) is used in conjunction with the monomer containing two or more olefinically unsaturated groups. Preferred monofunctional monomers

include benzyl methacrylate and 3,3,5-trimethylcyclohexyl methacrylate. When used, the monofunctional monomer is preferably used in amounts of from about 5 to about 40 weight per cent of the monomer portion of the formulation.

The composition of the invention may contain fillers such as silica, powdered glass, powdered quartz, or the like, which are conventional in the art.

For composite applications, the filler preferably has a volume average particle size below 15 microns, and preferably, below 5 microns. Thirty per cent of the filler particles, and preferably 70 to 100 per cent, have a size below 5 microns. The filler is preferably employed in an amount within the range of from about 35 to about 78 volume per cent, based on the volume of the filler plus the polymerizable composition. Thus, the filler is preferably employed in relatively high proportions. A volume per cent of 35 to 78 corresponds approximately to 50 to 95 weight per cent of the dental restorative composition of the invention, depending on the specific gravity of the filler.

For composite applications, it is preferable to use hydrophobic, chemically durable fillers such as quartz and/or a particular heat-treated barium or strontium glass. Such hydophobic fillers will absorb less than 0.1 weight per cent water (prior to addition of silane coupling agent) when exposed to normal ambient conditions. Water content of the filler is determined by a differential scanning calorimeter ("DSC"). The first departure from baseline in a DSC scan is caused by the presence of water. To determine the amount present, the area under the peak is determined and normalized relative to the weight of the sample.

The barium or strontium glass that may be employed as the filler is selected for chemical durability, as is evidenced by resistance to leaching in an aqueous environment. Such glasses are substantially free of alkali metal oxides, and are single phase glasses. If the mole per cent of barium or strontium oxide exceeds a certain point, the glass becomes two-phased. This proportion can vary, depending upon the presence and proportion of other metal oxides in the glass. For one preferred type of glass that is composed of oxides of barium, silicon, boron, and aluminum, the upper limit for a single phase glass is about 20 mole per cent barium oxide. One preferred glass for use in the invention has the following composition:

$SiO_2$ - 67 mole per cent
$BaO$ - 16.4 mole per cent
$B_2O_3$ - 10 mole per cent
$Al_2O_3$ - 6.6 mole per cent

The essential ingredients in the glass are the oxides of barium and/or strontium and silicon. Oxides of other metals such as aluminum and boron may also be present so long as such oxides do not detract from the chemical durability of the glass. Thus, significant quantities of alkali metal oxides should be avoided because, as is well known, alkali metal ions are quite soluble in aqueous media, and therefore will reduce the chemical durability of the glass. The minimum barium and/or strontium content of the glass is preferably that which is sufficient to impart X-ray opacity to the glass.

The preferred barium and/or strontium glass powder is acid washed and then subjected to a heat treatment to enhance its resistance to attack by water. The procedures are the following:

The acid-washing treatment to which the glass powder is subjected is carried out by known procedures. For instance, a mixture of 1 part (by weight) of glass powder, 1 part of 37 per cent aqueous hydrochloric acid, and 1 part of de-ionized water is stirred at room temperature for 45 minutes, filtered, and rinsed with de-ionized water until the pH of the filtrate is the same as the rinse water. The powder is then dried at about 50°C. overnight in a forced air oven. The acid wash is used to remove metal impurities from the glass and to reduce the amount of leachable barium or strontium from the surface of the glass.

The acid-washed glass powder is subjected to a heat treatment to reduce the affinity of the glass powder for water. This heat treatment is carried out at an elevated temperature below the sintering temperature of the glass powder (the sintering temperature can be determined by known procedures, as by thermo-mechanical analysis "TMA"), but high enough to cause a significant reduction in the specific surface area of the glass powder, as measured by known procedures such as by a "Quantasorb" B.E.T. surface area analyzer. The reduction in specific surface area will usually be at least 50 per cent (i.e., the surface area of the heat treated glass powder will be less than about one-half that of the untreated powder), up to 80 to 90 per cent, or even more in some cases. The heat treatment time is not at all critical in that it need be carried out only for the minimum time needed to heat all the powder to the desired temperature. Apparently the effect of the heat on the glass powder is quite rapid, and all that is required is to bring all of the mass of powder up to the desired temperature. However, since the glass powder is an excellent heat insulator, this can take several hours for masses of powder wherein the heat must

travel through a significant thickness of powder to heat all of the glass to the desired temperature.

As is known in the art, a silane coupling agent can be employed to enhance the bond between the filler and the resin. Such coupling agents include gamma-methacryloxypropyltrimethoxysilane.

In many cases, such as in composite formulations, it is desirable to include a small percentage of colloidal silica in the composition in order to adjust the viscosity and the handling characteristics of the paste. For instance, from about 2 to about 25 weight per cent of colloidal silica, based on weight of the entire composite, is beneficial.

The colloidal silica is preferably treated with a silane coupling agent such as gamma-methacryloxypropyl-trimethoxysilicone ("A-174"). After such treatment, the silica should be protected from ambient moisture because it may absorb up to about 1 weight per cent of water from the atmosphere, as measured by DSC.

The major novelty of the invention is the use of an acylphosphine oxide as the photosensitizing catalyst. The acylphosphine oxides employed in the invention can be represented by Formula I:

$$(R)_2\text{--}P(O)\text{--}CO\text{--}R^1 \qquad (I)$$

wherein each R individually is a hydrocarbyl group such as alkyl, cycloalkyl, aryl, aralkyl, which can be halo-, alkyl-, or alkoxy-substituted, or the two R groups can be joined to form a ring along with the phosphorus atom, and wherein $R^1$ is a hydrocarbyl group, an S, O, or N containing five- or six-membered heterocyclic group, or

the group $-Z-CO-P(O)-(R)_2$ wherein each R individually has the meaning set forth above, and wherein Z represents a divalent hydrocarbyl group such as alkylene or phenylene of from 2 to 6 carbon atoms. Usually the R groups will have from 1 to 8 carbon atoms, and the $R^1$ group will have from 2 to 18 carbon atoms.

The acylphospine oxides employed in the invention can be produced by reacting an acid halide of the formula:

$$R^1-CO-X \qquad (II)$$

wherein $R^1$ is as described above and wherein X represents Cl or Br, with a phosphite of the formula:

$$(R)_2--P--OR^2 \qquad (III)$$

wherein each R individually is as described above, and wherein $R^2$ is a hydrocarbon group such as $C_1$ to $C_6$ alkyl or cycloalkyl of from 5 to 6 carbon atoms. A detailed description of the process for making the acylphosphine oxides is found in Hesse et al., U.S. Patent No. 4,265,723, referred to above.

The preferred acylphosphine oxides for use in the invention are those in which the R variables are phenyl or lower alkyl- or lower alkoxy-substituted phenyl, and wherein $R^1$ is phenyl or lower alkyl- or lower alkoxy-substituted phenyl. (By lower alkyl and lower alkoxy is meant such groups having from 1 to 4 carbon atoms.) The preferred acylphosphine oxide is 2,4,6-trimethylbenzoyldiphenylphosphine oxide.

It is preferable to employ the acylphosphine oxide in combination with a tertiary amine reducing agent.

Illustrative tertiary amines include ethyl 4-(N,N-dimethylamino)benzoate and N,N-dimethylaminoethyl methacrylate. The catalysts are employed in catalytically effective amounts, such as from about 0.1 to about 5 weight per cent (based on weight of olefinically unsaturated compound) of the acylphosphine oxide plus from about 0.1 to about 5 weight per cent (again, based on the weight of the olefinically unsaturated compound) of the tertiary amine.

The catalyst system is activated by exposure to light of a wave length of from about 250 nm to about 700 nm. Preferably, the wave length is in the visible light region (about 380 nm to 550 nm). The visible light sources ordinarily used for visible light-cured dental composites can be used with this catalyst system. The examples below illustrate one such light source and the conditions under which it is used.

The examples set forth below illustrate photopolymerizable compositions used in the invention. The following monomers were used in the Examples:

Ethoxylated bisphenol-A dimethacrylate ("EBDM")

Triethylene glycol dimethacrylate ("TEGDM")

1,6-hexamethylene glycol dimethacrylate ("HXDDM")

2,2'-propane bis[3-(4-phenoxy)-2-hydroxypropyl-1 methacrylate] ("Bis-GMA")

Benzyl methacrylate ("BMA")

In the Examples and Control Examples, the ingredients were mixed, de-gassed to insure bubble-free cured copositions, and cured by exposure to a commecial light source for photoinitiated reactions. For the samples without filler, the ingredients were simply mixed and de-gassed in a vacuum oven at room temperature for about 30 minutes, and were then exposed to a Translux commercial light source for 40 seconds. The distance between the source and the sample was about 0.5 to 1 inch. The samples were held in a glass-bottomed Teflon mold 2.5 millimeters deep during exposure to the light, and were aged at 37°C. in water for 24 hours before testing. For the samples that contained filler, the ingredients were mixed at room temperature and were vacuum degassed in a vacuum oven at room temperature for about 30 minutes, and were cured by exposure of both sides to a Translux or Fotofil commercial light source for 60 seconds, with the sample being held 4 to 5 inches away from the source. The Teflon mold was used, with glass slides on both the top and bottom.

Examples 1-8 and Control Examples 1-2

Unfilled resin samples, illustrative of bonding agent and pit and fissure sealant formulations, having the formulations displayed below in Tables I and III, were cured by the procedure set forth above, and were tested for hardness on the bottoms (i.e., the surfaces at the bottom of the mold and away from the light source). The formulations are displayed in Tables I and III and the hardness data are displayed in Tables II and IV:

0173567

TABLE I

| Compositions | Parts, by weight Control 1 | Example 1 | Example 2 | Example 3 | Example 4 |
|---|---|---|---|---|---|
| EBDM | 80 | 80 | 80 | 80 | 80 |
| HXDDM | 5 | 5 | 5 | 5 | 5 |
| BMA | 15 | 15 | 15 | 15 | 15 |
| Camphoroquione | 0.28 | -- | -- | -- | -- |
| Benzil | 0.12 | -- | -- | -- | -- |
| Ethyl 4-(N,N-dimethylamino)-benzoate | 1.2 | 1.5 | 1.5 | 1.5 | 1.5 |
| Cyasorb UV-9 | 1.016 | -- | -- | -- | -- |
| 2,4,6-trimethyl-benzoyldiphenyl-phosphine oxide | -- | 0.5 | 1.0 | 2.5 | 2.25 |

TABLE II

| Samples | Hardness(1) |
|---|---|
| Control 1 | 43 |
| Example 1 | 28 |
| Example 2 | 29 |
| Example 3 | 47 |
| Example 4 | 34 |

(1) ASTM E-384. Samples tested after aging 1 hour in 37°C. water. Samples were exposed on one side only for 40 seconds with a Translux light.

TABLE III

| Compositions | Parts, by weight Control 2 | Example 5 | Example 6 | Example 7 | Example 8 |
|---|---|---|---|---|---|
| Bis-GMA | 45 | 45 | 45 | 45 | 45 |
| TEGDM | 55 | 55 | 55 | 55 | 55 |
| Camphoroquinone | 0.27 | -- | -- | -- | -- |
| Benzil | 0.11 | -- | -- | -- | -- |
| Ethyl 4-(N,N-dimethylamino)benzoate | 1.2 | 1.2 | 1.2 | 1.2 | 1.2 |
| 2,4,6-trimethylbenzoyldiphenylphosphine oxide | -- | 0.38 | 0.5 | 0.8 | 0.2 |

TABLE IV

| Samples | Shore 15T Hardness[1] |
|---|---|
| Control 2 | 50.2 ± 1.51 |
| Example 5 | 43.6 ± 1.86 |
| Example 6 | 44.7 ± 2.23 |
| Example 7 | 41.4 ± 1.53 |
| Example 8 | 33.4 ± 2.13 |

(1) Samples exposed on one side only for 40 seconds with Translux light, and then aged 1 hour in 37°C water.

Examples 9-10 and Control Examples 3-4

Filled resin systems, illustrative of composite restorative formulations, having the formulations displayed below in Tables V and VII, were cured by the procedure set forth above, and were tested for certain representative physical properties, with the results being displayed in Tables VI and VIII:

Table V

| COMPOSITION | PARTS BY WEIGHT | |
|---|---|---|
| | CONTROL 3 | EXAMPLE 9 |
| Control 1 Resin[1] | 16 | - |
| Example 1 Resin[1] | - | 16 |
| Barium glass[2] | 70 | 70 |
| Colloidal Silica[3] | 14 | 14 |

---

(1) Formulation given in Table I.

(2) Barium glass powder, 13μ, maximum size.

(3) OX-50 colloidal silica.

---

Table VI

| PROPERTIES | CONTROL 3 | EXAMPLE 9 |
|---|---|---|
| Diametral Tensile (MPa) | 66.7 ± 6.7 | 70.2 ± 10.1 |
| Compression Strength (MPa) | 344.9 ± 37.5 | 301.1 ± 58.5 |
| Flexural Strength (MPa) | 121.9 ± 15.6 | 122.5 ± 23.9 |
| Flexural Modulus (MPa) | 17765 ± 1131 | 17292 ± 1227 |
| Hardness (F Scale) | 102.1 ± 1.0 | 101.8 ± 1.3 |

Table VII

| COMPOSITION | PARTS BY WEIGHT | |
|---|---|---|
| | CONTROL 4 | EXAMPLE 10 |
| Control 2 Resin[1] | 48 | - |
| Example 5 Resin[1] | - | 48 |
| Colloidal Silica[2] | 52 | 52 |

(1) Formulation given above in Table III.

(2) OX-50 colloidal silica

Table VII

| PROPERTIES | CONTROL 4 | EXAMPLE 10 |
|---|---|---|
| Diametral Tensile (MPa) | 26.5 ± 3.8 | 24.4 ± 6.7 |
| Compression Strength (MPa) | 307 ± 42 | 290 ± 53 |

The test procedures that were used to evaluate Examples 9 and 10 and Control Examples 3 and 4 were the following: Diametral tensile strength - ADA Specification No. 27, Paragraph 4.3.7, JADA 94, 1191 (1977).

Flexural Strength and Modulus - Three-point bend test. A molded beam, 1/16 inch in thickness, is supported across a gap of one inch and a stressing force is applied to the center of the beam midway between the supports, until the beam breaks. The speed of the Instron testing machine used is 0.05 cm/min.

Compression Strength - a right cylinder of the polymerized dental restorative, after conditioning in a 37°C. water bath for 24 hours, is stressed in the compression mode by an Instron tester (at a speed of 0.5 cm/in), parallel to its long axis, until failure occurs.

Hardness - Rockwell F scale used.

The photopolymerizable compositions described above are applied to a prepared tooth in the customary way. For instance, when the composition is used as a pit and fissure sealant on the occlusal surface of a tooth, the surface is first preferably etched with, e. g., 30-40 weight per cent aqueous phosphoric acid, rinsed, and thoroughly dried prior to the application of the photopolymerizable composition. When the composition is used as a composite to restore a carious lesion, the carious material is removed in the usual manner, the cavity is cleaned and dried, and the photopolymerizable composition is then applied in the usual manner. A bonding agent may be applied before the composite.

CLAIMS



1. A photopolymerizable composition comprising at least one olefinically unsaturated monomer and a catalyst comprising an acylphosphine oxide for use in treating teeth by its application to a prepared tooth.

2. The composition of Claim 1, wherein the monomer includes at least one acrylic or methacrylic ester.

3. The composition of Claim 1 or Claim 2, wherein the catalyst comprises an acylphosphine oxide of the formula:

$$(R)_2\text{--P(O)--CO--}R^1$$

wherein each R individually is a hydrocarbyl group, which can be halo, alkyl, or alkoxy substituted, and wherein $R^1$ represents a hydrocarbyl group, an S,O, or N containing five - or six membered heterocyclic ring, or the group $-Z-CO-P(O)-(R)_2$ wherein R is as defined above, and Z represents a divalent hydrocarbyl group.

4. The composition of any one of claims 1 to 3,wherein the catalyst also contains a tertiary amine.

5. The composition of any one of claims 1 to 4, wherein the acylphosphine oxide is 2,4,6-trimethylbenzoyldiphenylphosphine oxide.

6. The composition of any one of claims 1 to 5, wherein the composition contains a finely divided inorganic filler.

7. The use of the composition of any one of claims 1 to 6 for treating teeth by its application to a prepared tooth.

8. A method of making the composition of any one of claim 1 to 6 comprising mixing together the components thereof.